# EUROPÄISCHE PATENTSCHRIFT

(11) **EP 0 696 899 B1**
(45) Veröffentlichungstag und Bekanntmachung des Hinweises auf die Patenterteilung: **05.06.2002**
(21) Anmeldenummer: 95909622.3
(22) Anmeldetag: 07.02.1995
(51) Int. Cl.: A61B 17/00

(54) **EXTRAKTIONSBEUTEL FÜR DIE ENDOSKOPISCHE CHIRURGIE**
EXTRACTION BAG FOR ENDOSCOPIC SURGERY
SAC D'EXTRACTION POUR CHIRURGIE ENDOSCOPIQUE

(30) Priorität: 07.02.1994 DE 4403567
(43) Veröffentlichungstag der Anmeldung: 21.02.1996
(73) Patentinhaber: Karl Storz GmbH & Co. KG, 78532 Tuttlingen (DE)
(72) Erfinder: CUSCHIERI, Alfred, Dundee DD1 9SY (GB)
(74) Vertreter: Münich, Wilhelm, Dr.
(86) Internationale Anmeldenummer: DE9500157
(87) Internationale Veröffentlichungsnummer: WO9520915

(56) Entgegenhaltungen:
- WO-A-92/11816
- WO-A-93/15671
- WO-A-93/15675
- WO-A-94/26179
- DE-U- 9 212 714
- US-A- 5 279 548

## Beschreibung

### Technisches Gebiet

Die Erfindung bezieht sich auf eine Vorrichtung zur Durchführung endoskopischer Operationen mit einer flexiblen Hülle, die abgetrennte Teile aufnimmt, gemäß dem ersten Teil des Patentanspruchs 1.

Die endoskopische Operationstechnik erlaubt es dem behandelnden Arzt, unter Beobachtung der zu behandelnden intrakorporalen Stellen die verschiedensten Eingriffe im lebenden Körper auszuführen.

### Technisches Gebiet

Häufig soll bei einem endoskopischen Eingriff eine körpereigene Probe abgetrennt und entnommen werden. Hierzu sind die verschiedensten Schneidinstrumente für das Abtrennen und Entnehmen körpereigener Proben, wie beispielsweise von Gewebe, von Organen oder Organteilen, Tumoren, Blasen-, Gallen-, Nierensteinen oder ähnliches vorgeschlagen worden.

Ein gravierendes Problem besteht dabei darin, daß durch den Entnahmevorgang eine Infizierung des umliegenden Körperbereiches, bspw. des umliegenden gesunden Gewebes entlang des Durchführungskanals durch die Körperdecke, nicht mit Sicherheit ausgeschlossen werden kann. Die Gefahr einer Infizierung tritt insbesondere beim Abtrennen und Entfernen von bösartigen Geschwüren auf.

Grundsätzlich ist diese Gefahr dadurch zu vermeiden, daß die zu entnehmende Probe nach Beendigung des Abtrennvorgangs in ein in den Körper eingebrachtes Behältnis eingeführt wird, dieses verschlossen und zusammen mit der abgetrennten Probe durch vorhandene oder für den Eingriff geschaffene Durchgänge aus dem Körper gebracht wird.

Aus der EP 0 507 588 A1 ist ein medizinisches endoskopisches Instrument bekannt, das an seinem distalen Ende einen verschließbaren Sack aufweist, der durch eine Körperöffnung ins Körperinnere gebracht werden kann und dort im geöffneten Zustand das Einbringen abgetrennter Körperproben gestattet. Durch Betätigung eines am proximalen Ende vorgesehenen Bedienmechanismus ist dieser Sack bzw. Beutel verschließbar. Der geschlossene Sack kann dann aus dem Körperinneren entnommen werden, ohne daß die Gefahr einer Infizierung von Körperteilen durch Verschleppung von Krankheitskeimen und/oder Krebszellen etc. besteht.

Aus der WO 92/14407 ist ein Extraktionsbeutel für die endoskopische Chirurgie bekannt, der an einem für das Einbringen und die Entnahme in bzw. aus dem Körperinneren vorgesehenen Instrument angebracht ist und über eine wiederverschließbare Öffnung verfügt.

Ferner geht aus der WO 92/11816 ein Behältnis hervor, das entweder durch eine natürliche oder künstliche Körperöffnung in das Körperinnere gebracht werden kann und dort als ein sackförmiger Container für die Aufnahme von Gewebe, Organe oder Organteilen, Tumoren, steinförmige Ablagerungen wie beispielsweise Nierensteine oder andere körpereigene Proben, die durch diverse medizinische Hilfsmittel behandelt worden sind, dient. Auch in diesem Fall handelt es sich um einen mit einer einzigen Öffnung versehenen Sack, der selbst aus einem robusten Material besteht, so daß in ihm in einem gewissen Umfange Verkleinerungsvorgänge an den in dem Sack eingebrachten Gegenstände vorgenommen werden können.

Schließlich ist aus der DE 42 20 785 A1 ein Laparoskopiebeutel bekannt, der eine trichterförmige Gestalt aufweist und ein Ansammeln abgetrennter Körperteile innerhalb des schmalen, trichterförmig zulaufenden Bereich vorsieht. Diese Ausführungsform verfügt ebenso, wie die vorstehend angeführten Extraktionsbeutel nur über eine einzige Öffnung.

Zwar wird mit den vorstehend vorgestellten, bekannten Ausführungsformen für Extraktionsbeutel das Ziel erreicht, im Körper abgetrennte Proben, die möglicherweise für den Körper schädliche Stoffe bzw. Zellen enthalten, unter Ausschaltung der Gefahr einer Infizierung der umliegenden Körperteile durch Verschleppung von Keimen, bösartigen Zellen, Bakterien etc. aus dem Körperinneren zu entnehmen. Dennoch haben diese bekannten Behältnisse gewisse Nachteile:

Zum einen ist die intrakorporale Befüllung der vorgeschlagenen Extraktionsbeutel kompliziert: Die bekannten Beutel weisen nämlich nur eine einzige Öffnung auf, in die die abgetrennten Probenstücke mit einem dafür vorgesehenen Instrumentarium "hineingeschaufelt" werden müssen. Dieser Vorgang benötigt einen gewissen Bewegungsfreiraum, der jedoch intrakorporal, d.h. in einer Körperhöhle nur selten zur Verfügung steht.

Grundsätzlich wird bei der Entnahme von Körperproben mit Hilfe der vorgenannten Extraktionsbeutel die maximale Größe der zu entnehmenden Körperprobe durch die Dimensionierung der Körperöffnung begrenzt, durch die das Instrumentarium mit dem Extraktionsbeutel geführt wird. Werden Proben im Körperinneren abgetrennt, die größer als die Öffnung sind, ist es erforderlich, diese so zu zerkleinern, daß sie, nachdem sie in den Beutel eingebracht sind, durch die Körperöffnung nach außen entnommen werden können. Zwar ist es möglich, daß der Zerkleinerungsvorgang bereits innerhalb des Extraktionsbeutels vorgenommen werden kann, doch ist selbst bei vorsichtiger Vorgehensweise nicht ausgeschlossen, daß den Körper schädigende Stoffe, die während der Probenzerkleinerung aus dem Probenmaterial austreten können, in den umliegenden intrakorporalen Bereich eindringen, da die bekannten Extraktionsbeutel keine zuverlässigen Vorkehrungen aufweisen, die die Beutelöffnung dicht gegen ein in den Beutel eingebrachtes Zerkleinerungsinstrument abschließen.

Eine Kontamination des gesunden umliegenden Gewebes ist auf diese Weise bereits innerhalb der Körpers nicht ausgeschlossen.

Alle bekannten Extraktionsbeutel sind ausschließlich als Containereinheiten zu benutzten, die innerhalb des Körpers befüllt und verschlossen werden. Sie bieten nämlich keine Möglichkeit, Manipulationen an den abgetrennten Proben innerhalb des verschlossenen Beutels mit Hilfe geeigneter Instrumente vorzunehmen.

Eine Vorrichtung mit den Merkmalen des ersten Teils des Anspruchs 1 ist aus WO-A-93/15671 bekannt.

### Darstellung der Erfindung

Der Erfindung liegt daher die Aufgabe zugrunde, eine Vorrichtung zur Durchführung endoskopischer Operationen innerhalb einer intrakorporalen Hülle anzugeben, die die Möglichkeit von intrakorporalen Infektionen aufgrund abgetrennter Körperproben während des Abschneidens, der Zerkleinerung sowie der Entnahme aus dem Körper praktisch vollständig ausschließt.

Desweiteren soll die erfindungsgemäße Vorrichtung die Möglichkeit bieten, die in das Innere des Körpers eingebrachte Hülle, z.B durch Insufflation, derart zu manipulieren, daß Bewegungsfreiraum für die Probenbearbeitung innerhalb des Körpers geschafft werden kann. In diesem Zusammenhang sollte die erfindungsgemäße Hülle ebenso die Möglichkeit bieten, die Handhabung mit dem notwendigen Chirurgiebesteck zu vereinfachen, so daß die Bearbeitung von Körperproben in intrakorporalen Bereichen, an denen andere Druckverhältnisse herrschen können als im extrakorporalen Bereich, von außen möglichst ohne große Umstände durchgeführt werden kann.

Eine erfindungsgemäße Lösung dieser Aufgabe ist im Anspruch 1 angegeben. Weiterbildungen der Erfindung sind Gegenstand der Ansprüche 2 und folgende.

Der Erfindung liegt die Idee zugrunde, endoskopische Operationen innerhalb einer intrakorporalen Hülle vorzunehmen, die mindestens zwei Öffnungen aufweist, und ballonartig auf eine vordefinierte Form aufblasbar bzw. insufflierbar ist.

Die auf diese Weise erfindungsgemäß ausgestaltete Hülle wird in aller Regel nach der Abtrennung der Körperprobe, beispielsweise eines Gewebe- bzw. Organteils in den entsprechenden Körperbereich, beispielsweise in den Bauchraum (Abdomen) eingebracht. Dabei ragt die schlauchförmig verlängerte Öffnung über die Einführhülse aus dem Körper heraus. Ein von außen in den Bauchraum eingeführtes Greifinstrument wird durch diese Öffnung der Hülle derart geführt, daß das Greifinstrument die abgetrennte Körperprobe in die Hülle selbst durch die zweite größere Öffnung, durch die das Greifinstrument herausragt, hineinzieht.

Dabei können die Öffnungen der Hülle so bemessen sein, daß die kleinere Öffnung den Durchtritt beispielsweise eines Greifinstrumentes erlaubt und die größere Öffnung das Einbringen von Organen bzw. Organteilen oder ähnlichen abgetrennten Teilen in die Hülle ermöglicht. Letzter Öffnungsteil ist vorzugsweise über einen zweiten Einstich nach außen zu bringen und anschließend mit dem entsprechend dafür vorgesehenen Abbindelement zu verschließen. Nach dem Verschließen wird der Hüllenteil wieder in den Körper hineingezogen und das Gewebeteil entsprechend bearbeitet. Die kleinere Öffnung der erfindungsgemäßen Hülle liegt in bevorzugterweise derart eng an dem, durch die Öffnung durchgeführten Instrument an oder ist zusätzlich durch ein Abbindeelement dicht an das Instrument, bzw. die Einführhülse angebunden, so daß ein Stoffaustausch zwischen Hülleninnenraum und dem übrigen intrakorporalen Raum unmöglich ist.

Die Hülle besteht erfindungsgemäß aus einem dehnbaren Material und ist mit geeigneten Hilfsmitteln, wie beispielsweise sogenannten Insufflatoren, insufflierbar, d.h. aufblasbar. Auf diese Weise wird innerhalb des Körperinneren ein abgeschlossener Raum geschaffen, der es gestattet weitere Bearbeitungsschritte isoliert von dem gesunden Körperumfeld vorzunehmen. Somit können kontrollierte Zerkleinerungen der Körperprobe mit Hilfe von Schneidwerkzeugen unter Beobachtung mittels Endoskope, die bspw. über die andere Hüllenöffnung in den Hüllenraum hineinragen, durchgeführt werden.

Ist der Zerkleinerungsvorgang abgeschlossen, so ist die Hülle beispielsweise durch die zweite Öffnung durch die Bauchdecke komplett zu entnehmen.

Mit der erfindungsgemäßen Vorrichtung zur Durchführung endoskopischer Operationen im intrakorporalen Bereich innerhalb einer Hülle ist es somit erstmalig möglich, die zur weiteren Untersuchung abgetrennte Körperprobe bzw. das Organteil mittels eines zusätzlichen Schneideinstruments im Körperinneren in kontrollierter Weise zu zerkleinern, so daß sie zum einen mit der Hülle durch eine Körperöffnung nach außen gebracht werden können, ohne die schmale Körperöffnung stark zu dehnen und zum anderen zur hystologischen Untersuchung wieder in ihrer vorherigen Gestalt zusammengesetzt werden können.

Mit der erfindungsgemäßen Vorrichtung ist somit ein medizinisches Werkzeug geschaffen worden, mit Hilfe dessen innerhalb des Körpers ein abgeschlossener Raum geschaffen werden kann, innerhalb dem chirurgische, vorzugsweise endoskopische, Bearbeitungsschritte vorgenommen werden können.

Ferner sieht ein weiterer erfindungsgemäßer Aspekt eine Ausgestaltung der vorstehend beschriebenen Hülle vor, bei dem die Hülle nicht als ganzes in den intrakorporalen Bereich einbringbar ist, sondern nur teilweise, so daß die Hülle durch eine Körperöffnung -auch größerer Ausdehnung - ragend, einen intra- und extrakorporalen Hüllenbereich bildet. Mit Hilfe dieser erfindungsgemäßen Ausführungsform ist es möglich, die intrakorporalen Druckbedingungen auch innerhalb des extrakorporalen Hüllenbereichs aufrechtzuhalten, sofern die in diesem Bereich vorgesehenen Öffnungen dicht gegenüber der Außenatmosphäre sind. Mit Hilfe selbstdichtender Öffnungen ist das Hantieren mit Instrumentarien, die in die Hülle eingeführt werden können, oder auch direkt mit der Hand erheblich zu vereinfachen. Somit sind beispielsweise nach außen durch die Körperöffnung hindurchgeführte Organe bzw. Organteile, die mit diversen anderen inneren Körperteilen noch verbunden sind, innerhalb des extrakorporalen Hüllenbereiches in gewünschter Weise zu manipulieren. Auf den Einsatz von Endoskopen, die trotz fortgeschrittener Technik einen nur eng begrenzten Sichtbereich gestatten, kann somit im extrakorporalen Bereich der Hülle verzichtet werden.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: eine schematische Seitenansicht einer erfindungsgemäßen Hülle,
- Fig. 2a, b, c, d: eine schematische Bildsequenz der Handhabungsweise einerr erfindungsgemäßen Hülle, und
- Fig. 3: eine schematische Seitenansicht einer Ausführungsform der erfindungsgemäßen Hülle.

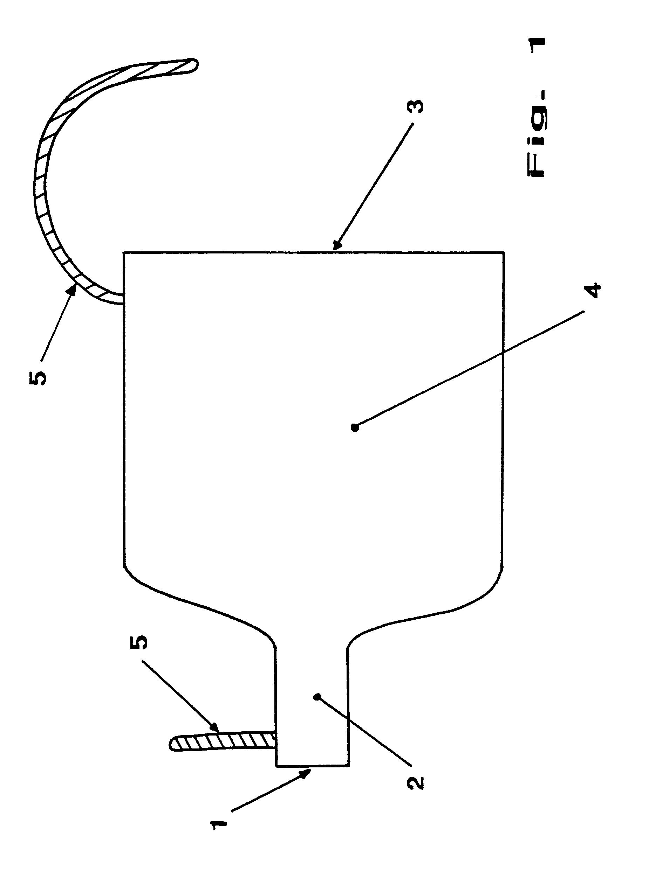

Eine besonders vorteilhafte Ausführungsform der erfindungsgemäßen Hülle ist in Figur 1 dargestellt. Die Hülle weist zwei Öffnungen auf, nämlich eine kleine Öffnung 1 und eine größere Öffnung 3. Die Größe der Öffnung 1 sowie des sich daran anschließenden schmalen, geradlinig verlaufenden Öffnungskanal 2 soll die Durchführung endoskopischer Instrumentarien, vorzugsweise durch die Einführhülse erlauben. Der Öffnungskanal 2 weitet sich bezogen auf die Gesamtlänge der erfindungsgemäßen Hülle innerhalb eines kurzen Bereiches auf den Durchmesser der größeren Hüllenöffnung 3 auf und bildet mit diesem größeren Durchmesser einen ebenso geradlinig verlaufenden Hüllenkanal 4, der länger ist als der Öffnungskanal 2.

Die größere Hüllenöffnung 3 ist derart dimensioniert, daß durch sie auch größere Organe bzw. Organteile in das innere des Hüllenkanals 4 gelangen können. Typischerweise beträgt der Hüllendurchmesser im Hüllenkanal 4 etwa 100 mm, mit einer Kanallänge von ca. 500 mm.

Ferner weist die erfindungsgemäße Hülle an ihren beiden Öffnungen 1 und 3 Abbindeelemente 5 auf. Diese Abbindeelemente 5 sind in bevorzugter Weise als bandförmige Schließvorrichtungen ausgebildet, die um die jeweilige Öffnung legbar die Öffnungen 1 bzw. 3 dichtend gegenüber dem Hüllenäußeren abschließen.

Die Hüllenform selbst weist eine Flaschenform auf, die es gestattet, körpereigene Proben in das Innere der Hüllen zu ziehen und dort zu bearbeiten. Die oftmals scharfkantigen endoskopischen Instrumentarien zur Zerkleinerung der abgetrennten Proben dürfen dabei die Hüllenwand nicht verletzen. Aus diesem Grund besteht die Hülle aus einem reißfesten Werkstoff, beispiels-weise Nylon. Darüberhinaus ist der Hüllenwerkstoff elastisch, leicht sterilisierbar und ein Teil der Hülle ist transparent, damit er für den medizinischen, endoskopischen Einsatz problemlos zu verwenden ist und die Möglichkeit der Insufflation gestattet.

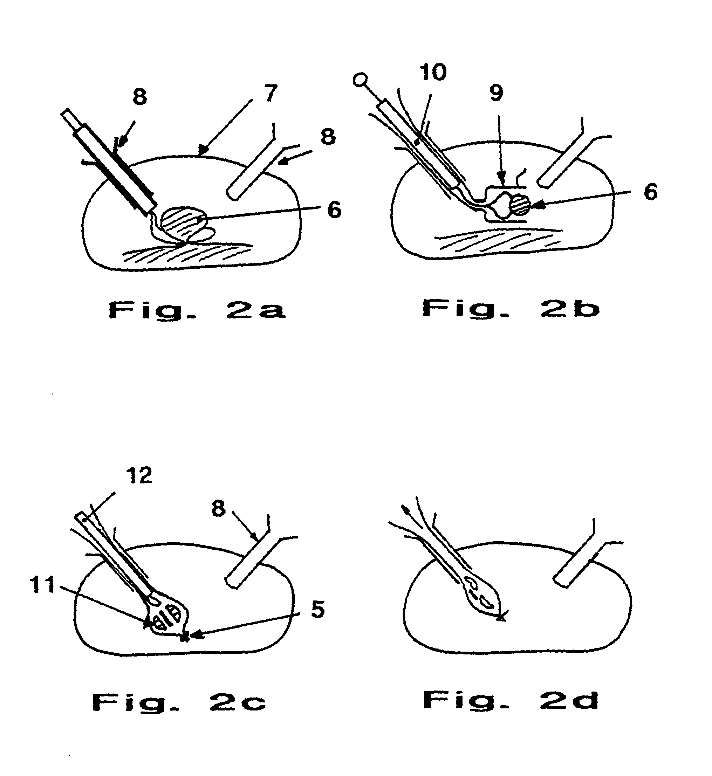

In den Figuren 2a bis 2d sind Bildsequenzen der typischen Anwendungsweise der erfindungsgemäßen Hülle dargestellt. Aus den Figuren geht ein Querschnitt durch die Bauchhöhle hervor, innerhalb der ein abzutrennendes Organteil 6 aus dem intrakorporalen Bereich entfernt werden soll. In vorteilhafter Weise sind durch die Bauchdecke 7 zwei künstliche Öffnungen eingebracht, durch die endoskopische Instrumentarien durch zwei Einführschäfte (Trokar-Schäfte) 8 in das Körperinnere eingeführt werden können.

In Figur 2a ist durch den linken Trokar-Schaft 8 ein endoskopisches Schneideinstrument eingebracht, das am distalen Ende über eine Schneidevorrichtung - in der Figur ist eine Schneidschlinge dargestellt - verfügt und das abzutrennende Organteil 6 vom übrigen Organ abschneidet.

In einem darauffolgenden Schritt, der in Figur 2b dargestellt ist, wird die erfindungsgemäße Hülle 9, die zunächst möglichst klein zusammengefaltet ist und somit durch einen der beiden Durchführungsschäfte geführt werden kann - bevorzugt wird die Hülle durch den dargestellten rechten Trokar-Schaft eingeführt, da durch den Linken Instrumentarien zum Fixieren des abgetrennten Körperteils eingebracht sind -, in das Innere des Körpers gebracht. Die Hülle wird innerhalb des Körpers mit Hilfe von geeigneten endoskopischen Hilfsmitteln aufgefaltet und insuffliert. In dem in Figur 2b dargestellten Zustand wird, unter endoskopischer Beobachtung ein Greifinstrument 10 durch die kleinere Öffnung der Hülle 9 geführt, dessen distales Ende das abgetrennte Organteil 6 erfaßt und durch geeignete Bedienung das Organteil in das innere der Hülle 9 hineinzieht. Hierzu wird in vorteilhafter Weise der schmale Hüllenkanal 2 durch den Trokar-Schaft hindurchgezogen, durch den das Greifinstrument 10 ragt. Dies verhindert bereits eine mögliche Kontamination mit dem umliegenden Gewebe.

Nachdem sich die abgetrennte Körperprobe im Inneren der Hülle 9 befindet, ist die größere Hüllenöffnung unter Verwendung geeigneter Hilfsmittel, die beispielsweise durch den linken Trokar-Schaft 8 in Figur 2c in den inkorporalen Bereich eingeführt werden können, mit Hilfe des Abbindeelementes 5 dichtend zu verschließen. Somit entsteht im Inneren des Körpers ein abgetrennter Raum, innerhalb dem mit Hilfe geeigneter Schneidevorrichtungen 12 die Probe derart zu zerkleinern ist, daß die Teilstücke 11 der Probe nach der Entnahme aus dem Körper zur histologischen Untersuchung gegenseitig wieder zusammengesetzt werden können.

Das Hüllenvolumen ist dadurch zu vergrößeren, daß der einseitig, dichtend abgeschlossene Hüllenbeutel von außen mit Hilfe eines geeigneten Zuführinstrumentes (nicht dargestellt) beispielsweise mit CO2-Gas insuffliert wird, damit der Bearbeitungsvorgang innerhalb des Beutels leichter erfolgen kann. Die erfindungsgemäße Hülle gewährleistet durch ihre Form, daß das Schneideelement die kleine Öffnung der Hülle 9 durchragt und diese gegenüber dem äußeren des intrakorporalen Körperbereiches abschließt. Somit ist ein weiterer isolierter Raum innerhalb des Körpers geschaffen, aus dem keine den übrigen Körperraum infizierende Stoffe austreten können. Nachdem das abgetrennte Organteil mit Hilfe des Schneideinstrumentes innerhalb der abgeschlossenen Hülle in geeigneter Form zerteilt wurde, wird das Schneideinstrument entnommen.

In Figur 2d wird sodann die erfindungsgemäße Hülle durch den rechten Trokar-Schaft 8 nach Außen gezogen.

Die erfindungsgemäße Hülle bietet eine leicht bedienbare Operationshilfe zur sicheren Entnahme von Körperproben, ohne dabei Gefahr zu laufen daß gesundes Gewebe während der Probenentnahme durch möglicherweiser bösartige Zellen kontaminiert werden.

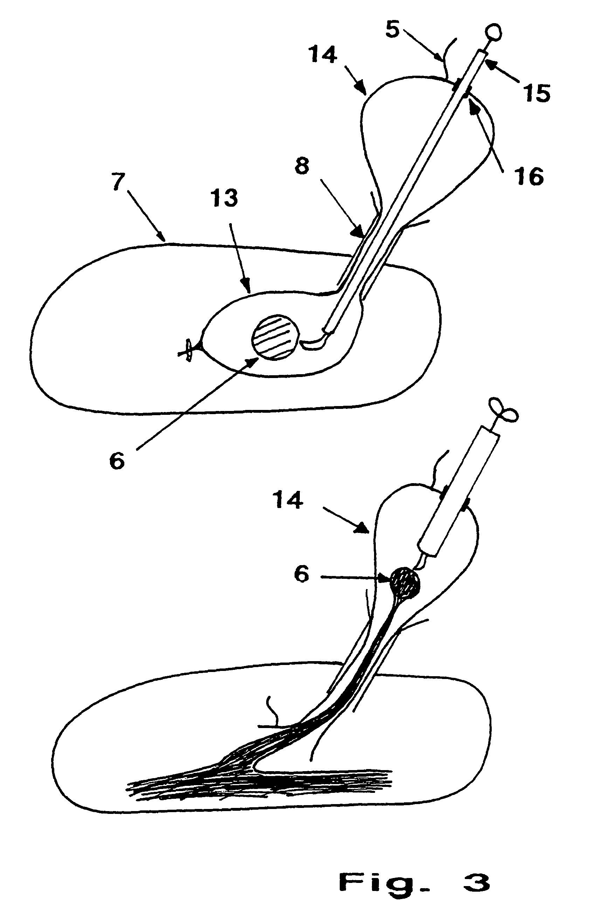

Aus Figur 3 geht eine leicht abgewandelte erfindungsgemäße Hüllenvorrichtung, die es gestattet die intrakorporalen Durckbedingungen im Rahmen einer externen Hülle nach außen zu bringen. In beiden Bilddarstellungen der Figur 3 ist ein Querschnitt durch eine Bauchdecke 7 dargestellt, der einen Durchführungskanal 8 aufweist, durch den teilweise eine Hülle in das Innere des Körpers gebracht worden ist. In der dargestellten Figur ist ein intra- 13 und ein extrakorporaler 14 Hüllenbereich zu sehen.

Der intrakorporale Hüllenbereich 13 enthält in der oberen Darstellung der Figur 3 eine bereits abgetrennte Körperprobe 6, die mit Hilfe der vorstehenden Vorgehensweise in das Innere der Hülle gelangt ist. Ein Schneideinstrument 15, das durch den Teil des extrakorporalen Hüllenbereiches 14 in den intrakorporalen Hüllenbereich 13 hineinragt, erlaubt die Zerkleinerung von Körperproben 6. Diese Operationstechnik ist bevorzugt in den Fällen anwendbar, in denen die Operationsstellen in Körperbereichen liegen, an denen andere Druckverhältnisse vorherrschen als im extrakorporalen Bereich. Ein typischer Anwendungsfall ist das Setzen von Darmteilverbindungen, sogenannten Anastomosen.

Die Öffnung 16 in dem extrakorporalen Hüllenbereich 14 ist dabei derart ausgebildet, daß zwischen dem eingeführten Instrument 15 und der Hüllenhaut kein Stoffaustausch stattfinden kann. Nur so kann gewährleistet werden, daß die Druckverhältnisse innerhalb der gesamten Hülle konstant gehalten werden können. Die Abdichtung erfolgt entweder durch ein extra vorzusehndes Abbindeelement 5 oder wird durch eine selbstdichtende Öffnung gewährleistet.

In der unteren Bilddarstellung in Figur 3 ist der Fall gezeigt, daß ein Teil einer zu bearbeitenden Körperprobe 6 in den extrakorporalen Hüllenbereich 14 verbracht ist, innerhalb dem es möglich ist ohne endoskopische Sichtgeräte die Operation vorzunehmen, weil ein Teil der Hülle transparent ist.

## Patentansprüche

1. Vorrichtung zur Durchführung endoskopischer Operationen, mit einer flexiblen Hüll (9), die abgetrennte Teile (6) aufnimmt, wobei die Hülle mindestens zwei Öffnungen (1, 3) aufweist, von denen wenigstens eine Öffnung distal und wenigstens eine andere Öffnung im proximalen Bereich angeordnet ist und die Hülle aus einem elastischen, sterilisierbaren, reißfesten Werkstoff, wie bspw. Nylon, besteht, wobei ein Teil der Hülle transparent bzw. durchsichtig ausgeführt ist,
**dadurch gekennzeichnet, daß** die Hülle ballonartig auf eine vordefinierte Form aufblasbar bzw. insufflierbar ist.

2. Vorrichtung nach Anspruch 1,
**dadurch gekennzeichnet, daß** die Öffnungen in der Hülle unterschiedliche Durchmesser aufweisen.

3. Vorrichtung nach Anspruch 1 oder 2,
**dadurch gekennzeichnet, daß** an jeder Öffnung ein Abbindelement (5) vorhanden ist.

4. Vorrichtung nach Anspruch 3,
**dadurch gekennzeichnet, daß** das Abbindeelement eine bandförmige Schließvorrichtung ist, die bei geschlossenem Zustand einen Stoffaustausch zwischen Hülleninnen- und -außenseite verhindert.

5. Vorrichtung nach Anspruch 3 oder 4,
**dadurch gekennzeichnet, daß** die Größe der Öffnungen derart bemessen ist, daß durch sie endoskopische Instrumente durchführbar sind, und daß die Abbindelemente derart anbringbar sind, daß die jeweilige Öffnung mit dem hindurchgeführten Instrument verschlossen ist.

6. Vorrichtung nach Anspruch 5,
**dadurch gekennzeichnet, daß** die Größe der distalen Öffnung der Hülle derart bemessen sind, daß sie das Einbringen von Organen oder Organteilen sowie auch von endoskopischen Hilfsmitteln in die Hülle erlaubt.

7. Vorrichtung nach einem der Ansprüche 1 bis 6,
**dadurch gekennzeichnet, daß** die Hülle eine Flaschenform aufweist, daß die kleinere Öffnung am Ende eines schmalen, geradlinig verlaufenden Öffnungskanals (2) vorgesehen ist, der den "Flaschenhals" bildet, und der sich, auf die Gesamtlänge der Hülle bezogen, innerhalb eines kurzen Übergangsbereich wenigstens auf den Durchmesser der größeren Hüllenöffnung aufweitet, und daß der Hüllenabschnitt mit dem größeren Durchmesser einen geradlinig verlaufenden Hüllenkanal (4) bildet, in dem die größere Öffnung vorgesehen ist, und der länger als der Öffnungskanal ist.

8. Vorrichtung nach Anspruch 7,
**dadurch gekennzeichnet, daß** der größere Hüllendurchmesser etwa 10 cm und die Länge dieses Hüllenabschnittes etwa 50 cm beträgt.

9. Vorrichtung nach einem der Ansprüche 1 bis 8,
**dadurch gekennzeichnet, daß** die Hülle, abgesehen von den Öffnungen, eine vollständig geschlossene Form aufweist.

10. Vorrichtung nach einem der Ansprüche 1 bis 9,
**dadurch gekennzeichnet, daß** die Hülle derart zusammenlegbar ist, daß sie durch einen kleinkalibrigen TrokarSchaft mit einem Durchmesser von ca. 12 mm durchführbar ist.

11. Vorrichtung nach einem der Ansprüche 1 bis 10, **dadurch gekennzeichnet, daß** die Öffnungen in der Hülle zunächst als echte Sollbruchstellen ausgeführt sind, die
sich durch eine mechanische Einwirkung bspw. einer Instrumentenspitze öffnen lassen.

12. Vorrichtung nach einem der Ansprüche 1 bis 11,
**dadurch gekennzeichnet, daß** die Hülle einen intrakorporalen Hüllenbereich aufweist, der im Gebranch durch eine Körperöffnung hindurchreicht und mit einem extrakorporalen Hüllenbereich verbunden ist.

13. Vorrichtung nach Anspruch 12,
**dadurch gekennzeichnet, daß** zumindest die Öffnungen im extrakorporalen Hüllenbereich selbstdichtend sind.

14. Vorrichtung nach einem der Ansprüche 12 und 13,
**dadurch gekennzeichnet, daß** die Hülle so ausgebildet ist, daß die Druckverhältnisse innerhalb des intrakorporalen und des extrakorporalen Hüllenbereiches gleich sind.

15. Vorrichtung nach einem der Ansprüche 1 bis 14,
**dadurch gekennzeichnet, daß** das Hüllenmaterial nur im sichtbaren Wellenlängenbereich transparent ist.

16. Vorrichtung nach einem der Ansprüche 12 bis 15,
**dadurch gekennzeichnet, daß** der extrakorporal verbleibende Hüllenbereich am Rand der Körperöffnung über ein Spannelement abdichtbar ist.

## Claims

1. Device for performing endoscopic surgery, comprising a flexible enclosure (9) receiving severed parts (6), with said enclosure presenting at least two openings (1, 3) whereof at least one is distally disposed and at least one other opening is arranged in the proximal region, and wherein said enclosure consists of an elastic sterilisable tear-resistant material such as nylon, with one part of said enclosure being configured to be transparent or translucent, respectively,
**characterised in that** said enclosure is adapted to be blown up or inflated, respectively to a predetermined shape in the manner of a balloon.

2. Device according to Claim 1,
**characterised in that** said openings in said enclosure present different diameters.

3. Device according to Claim 1 or 2,
**characterised in that** a tie element (5) is provided on each opening.

4. Device according to Claim 3,
**characterised in that** said tie element is a tape-shaped closing means that prevents an exchange of substances between the inside and the outside of said enclosure in the closed state.

5. Device according to Claim 3 or 4,
**characterised in that** the size of said openings is so dimensioned that endoscopic instruments may be passed therethrough, and that said tie elements may be fastened in such a way that the respective opening will be closed by the instrument introduced therethrough.

6. Device according to Claim 5,
**characterised in that** the size of said distal opening of the enclosure is so dimensioned that it permits the introduction of organs or parts of organs as well as of endoscopic means into said enclosure.

7. Device according to any of the Claims 1 to 6,
**characterised in that** said enclosure presents the shape of a bottle, that said smaller opening is provided on the end of a narrow straight opening duct (2) that forms the "bottle neck" and flares, relative to the overall length of said enclosure, within a short transitional region at least to the diameter of said wider enclosure opening, and that the enclosure section having the wider diameter forms a straight enclosure duct (4) in which said wider opening is provided and which is longer than the opening duct.

8. Device according to Claim 7,
**characterised in that** said wider enclosure diameter corresponds to roughly 10 cm and the length of this enclosure section amounts to 50 cm approximately.

9. Device according to any of the Claims 1 to 8,
**characterised in that** said enclosure, with the exception of said openings, presents a completely closed form.

10. Device according to Claim 9,
**characterised in that** said enclosure may be folded in such a way that it may be passed through a small-gauge trocar shaft having a diameter of roughly 12 mm.

11. Device according to any of the Claims 1 to 10,
**characterised in that** the openings in said enclosure are initially formed as genuine rated break points adapted to be opened by mechanical action or an instrument point, for instance.

12. Device according to any of the Claims 1 to 11,
**characterised in that** said enclosure comprises an intracorporeal enclosure region which, when in use, extends through a body opening and is connected to an extracorporeal enclosure region.

13. Device according to Claim 12,
**characterised in that** at least the openings in said extracorporeal enclosure region are self-sealing.

14. Device according to any of the Claims 12 and 13,
**characterised in that** said enclosure is so configured that the pressure situation inside said intracorporeal enclosure region is equal to the pressure in said extracorporeal enclosure region.

15. Device according to any of the Claims 1 to 14,
**characterised in that** said enclosure material is transparent only in the visible wavelength range.

16. Device according to any of the Claims 1 to 15,
**characterised in that** said enclosure region remaining outside the body is adapted to be sealed at the edge of said body opening via a tensioning element.

## Revendications

1. Dispositif de chirurgie endoscopique, comprenant une gaine flexible (9) à recevoir des partie détachées (6), ladite gaine présentant au moins deux ouvertures (1, 3), dont au moins une se trouve du côté distal pendant qu'au moins autre est disposée dans la zone proximale, et dans lequel ladite gaine consiste en un matériau élastique non déchirable stérilisable, comme le Nylon, à une partie de ladite gaine étant configurée de façon, qu'elle soit transparente ou respectivement translucide,
**caractérisé en ce que** ladite gaine est apte à être gonflée à une forme prédéterminée de la manière d'un ballon.

2. Dispositif selon la revendication 1,
**caractérisé en ce que** lesdites ouvertures dans ladite gaine présentent des diamètres différents.

3. Dispositif selon la revendication 1 or 2,
**caractérisé en ce qu'**un élément liant (5) est disposé sur chaque ouverture.

4. Dispositif selon la revendication 3,
**caractérisé en ce que** ledit élément liant est un moyen de fermeture en bande, qui empêche un échange de substances entre l'intérieur et l'extérieur de ladite gaine en état fermé.

5. Dispositif selon la revendication 3 ou 4,
**caractérisé en ce que** la taille desdites ouvertures est dimensionnée de façon que des instruments endoscopiques puissent y passer, et **en ce que** des éléments liants puissent s'attacher de façon, que l'ouverture respective soit fermée par l'instrument y introduit.

6. Dispositif selon la revendication 5,
**caractérisé en ce que** la taille de l'ouverture distale de la gaine est dimensionné de façon, qu'elle permette l'introduction des organes ou parties d'organes ainsi que des moyens endoscopiques dans ladite gaine.

7. Dispositif selon une quelconque des revendications 1 à 6,
**caractérisé en ce que** ladite gaine présente la forme d'une bouteille, **en ce que** ladite plus petite ouverture est muni, d'une extrémité, d'un passage d'ouverture (2) étroit en ligne droite, qui constitue un "col de bouteille" et s'évase, relativement à la longueur totale de ladite gaine, dans une courte zone de transition, au moins au diamètre de ladite plus grande ouverture de la gaine, et **en ce que** la partie de gaine au plus grand diamètre forme un canal de gaine (4) en ligne droit, dans lequel ladite plus grande ouverture est formée et qui est plus longue que le passage d'ouverture.

8. Dispositif selon la revendication 7,
**caractérisé en ce que** ledit plus grand diamètre de la gaine correspond environ à 10 cm et la longueur de cette partie de la gaine correspond à 50 cm environ.

9. Dispositif selon une quelconque des revendications 1 à 8,
**caractérisé en ce que** ladite gaine, à l'exception desdites ouvertures, a une forme complètement fermée.

10. Dispositif selon la revendication 9,
**caractérisé en ce que** ladite gaine est pliable de façon, qu'elle puisse passer à travers une tige de trocart à petit calibre, dont le diamètre est de 12 mm environ.

11. Dispositif selon une quelconque des revendications 1 à 10,
**caractérisé en ce que** les ouvertures dans ladite gaine sont d'abord formées comme points réellement destinés à la rupture à ouvrir par actionnement mécanique ou par la pointe d'un instrument, par exemple.

12. Dispositif selon une quelconque des revendications 1 à 11,
**caractérisé en ce que** ladite gaine comprend une zone de gaine intracorporelle, qui, en cas d'emploi, s'étend à travers l'ouverture du corps, en étant reliée à une zone de gaine extracorporelle.

13. Dispositif selon la revendication 12,
**caractérisé en ce qu'**au moins les ouvertures dans ladite zone de gaine extracorporelle sont prévues à une fermeture automatique.

14. Dispositif selon une quelconque des revendications 12 et 13,
**caractérisé en ce que** ladite gaine est configurée de façon, que l'état de pression à l'intérieur de la zone intracorporelle soit égale à la pression régnant dans la zone de gaine extracorporelle.

15. Dispositif selon une quelconque des revendications 1 à 14,
**caractérisé en ce que** le matériau de ladite gaine n'est transparent que dans la gamme de longueurs d'onde visible.

16. Dispositif selon une quelconque des revendications 1 à 15,
**caractérisé en ce que** ladite zone de gaine, qui reste à l'extérieur du corps, est apte à être fermée, de façon étanche, au bord de l'ouverture du corps via un élément tendeur.
